# EUROPEAN PATENT APPLICATION

(11) **EP 1 164 235 A2**
(43) Date of publication of application: **19.12.2001**
(21) Application number: 01305036.4
(22) Date of filing: 08.06.2001
(51) Int. Cl.: E05B 1/00, A61L 2/18

(54) **Handle**

(30) Priority: 09.06.2000 GB 0014155
(71) Applicant: Rentokil Initial Plc, West Sussex RH19 2JY (GB)
(72) Inventor: Smith, Colin, Haywards Heath, West Sussex RH17 6AD (GB)
(74) Representative: Darby, David Thomas

(57) **Abstract**

A handle which comprises: a hollow handle member the outer surface of which, in use, is contacted by the hand of an operator on actuation of the handle, the handle member being arranged to contain a cleaning agent and having means for discharging cleaning agent; a holder for the handle member, the handle member being mounted for rotation about its longitudinal axis relative to the holder, the discharging means of the handle member being arranged to allow cleaning agent to be discharged from the interior of the handle member to the exterior upon such relative rotation and/or to be discharged subsequent to such relative rotation; means for rotating the handle member about its longitudinal axis relative to the holder in response to the actuation of the handle; and means for distributing cleaning agent discharged by the discharging means over the outer surface of the handle member upon relative rotation of the handle member and the holder.

## Description

The invention relates to handles, especially to handles for use in toilets, washrooms and kitchens, and more especially to handles for lavatory cisterns.

Handles in such situations often constitute hygiene risks owing to the fact that they are liable to contamination, and contaminants such as harmful bacteria can be passed on to one or more subsequent users.

Handles aimed at overcoming such problems have previously been proposed (see EP 0 351 307 (Alazet) and CH 568 074 (Cadlini)) but there remains a need for a compact and reliable handle that will clean itself with the aim of reducing or removing contaminants, and which can replace an existing handle in a simple manner.

The invention provides a handle which comprises:
a hollow handle member the outer surface of which, in use, is contacted by the hand of an operator on actuation of the handle, the handle member being arranged to contain a cleaning agent and having means for discharging cleaning agent;
a holder for the handle member, the handle member being mounted for rotation about its longitudinal axis relative to the holder, the discharging means of the handle member being arranged to allow cleaning agent to be discharged from the interior of the handle member to the exterior upon such relative rotation and/or to be discharged subsequent to such relative rotation;
means for rotating the handle member about its longitudinal axis relative to the holder in response to the actuation of the handle; and
means for distributing cleaning agent discharged by the discharging means over the outer surface of the handle member upon relative rotation between the handle member and the holder.

With the handle of the invention, the holder can be mounted on an appliance (for example, a door or lavatory cistern) and the handle replace a conventional handle. Actuation of the handle, for example, to open the door or flush the lavatory cistern on which it is mounted, necessitates a user contacting the outer surface of the handle member. Upon such actuation, or immediately afterwards, the handle member, which contains a cleaning agent or disinfectant composition, for example, an antibacterial and/or antifungal agent is arranged to rotate. The discharging means of the handle member is arranged to discharge cleaning agent when the handle rotates and/or subsequently thereafter. Cleaning agent, which may have been discharged during rotation, and/or may have been discharged previously and absorbed or otherwise retained by the distributing means for subsequent use, is spread by the distributing means over the outer surface of the handle member as it rotates. Thus, it is possible to spread a thin film of cleaning agent over substantially the entire area of the handle that is contacted by a user and reduce or substantially remove any contaminants on the handle, with the consequential reduction in the risk of any such contaminants being passed to the next user.

Although especially useful for use as a lavatory cistern handle in toilets and washrooms, the handle of the invention could, of course, be used in other applications, such as door handles in a kitchen.

Advantageously, the holder comprises one or more supporting arms for the handle member, the or each arm extending longitudinally relative to the handle member. The means for distributing cleaning agent over the outer surface of the handle member is preferably located between one or more of the arms and the handle member. The distributing means may be in the form of a brush or a strip of absorbent felt or sponge material.

In one embodiment of the invention, the discharging means is in the form of one or more outlets in the handle member and the holder is arranged to open the or each outlet on rotation of the handle member relative to the holder. In such an arrangement, the or each outlet is preferably arranged to be opened by deformation of the region of the handle member in the area immediately surrounding the outlet and the holder is arranged to deform the said region on relative rotation of the handle member. When the holder comprises one or more arms, the arms may be provided with one or more protrusions spaced along the arms, each protrusion being positioned so as to engage the handle member in the region of one of the outlets when the handle member rotates relative to the holder to cause the said deformation. The outlets may be, for example, in the form of so-called "Bunsen slits".

In alternative embodiments of the invention, the discharging means of the handle member comprises collecting means for collecting cleaning agent on relative rotation of the handle member and the holder, and the discharging means is arranged to dispense the collected cleaning agent to the distributing means. The discharging means advantageously further comprises one or more ducts or conduits arranged to allow collected cleaning agent to pass through the handle member to one or more discharge ports on its outer surface. Preferably, the handle member is arranged to rotate from a parked position by one or more whole turns relative to the holder on actuation of the handle, and the or each discharge port is arranged to coincide with the distribution means in the said parked position.

The collecting means of the discharging means may comprise one or more receptacles, for example, spoon- or cup-shaped receptacles, on the inside of the handle member. In another arrangement, the collecting means may comprise one or more cavities in the handle member. In a further arrangement, the collecting means of the or each outlet may comprise one or more gutters on the inside of the handle member, the or each gutter being arranged to collect cleaning agent remaining on the inside surface of the handle member after relative rotation. With those arrangements, the discharging means can dispense a measured dose of the cleaning agent, the size of the dose being predetermined at least to some extent by the capacity and/or number of the receptacles, cavities or gutters provided.

The means for rotating the handle member in response to the actuation of the handle may comprise a clockwork, or an electric, motor.

The cleaning agent may be any suitable agent, for example, isopropyl alcohol, and may be in the form of a gel or a liquid.

Advantageously, the handle member is removable from the holder for refilling with cleaning agent or replacement with a new handle member containing cleaning agent. When it is to be refilled, the handle member may be provided with a non-return valve so arranged as to allow the handle member to be supplied with cleaning agent. A lockable end cap may be provided at one end of the handle member for covering the non-return valve.

Four embodiments of a handle constructed in accordance with the invention will now be described by way of example with reference to the accompanying drawings, in which
FIGURE 1 is a diagrammatic plan view of the handle according to a first embodiment of the invention;
FIGURE 2 is a diagrammatic front view of the handle of FIGURE 1;
FIGURE 3 is a diagrammatic section taken along the line III-III shown in Figure 2;
FIGURE 4 is a diagrammatic end view of the first embodiment of the handle looking in the direction of arrow V in Figure 3 showing an end cap in a closed position;
FIGURE 5 is a diagrammatic end view of the first embodiment of the handle looking in the direction of arrow V in Figure 3 with the end cap in an open position;
FIGURE 6 is a diagrammatic section through part of a handle according to a second embodiment of the invention;
FIGURES 7 and 8 are diagrammatic cross-sections through the part of the handle of Figure 6 at different times;
FIGURE 9 is a diagrammatic section through part of a handle according to a third embodiment of the invention;
FIGURES 10 and 11 are diagrammatic cross-sections through the part of the handle of Figure 9 at different times;
FIGURE 12 shows, diagrammatically and enlarged, a detail of the handle of Figures 9 to 11;
FIGURE 13 is a diagrammatic section through part of a handle according to a fourth embodiment of the invention; and
FIGURE 14 is a diagrammatic cross-section through the part of the handle of Figure 13.

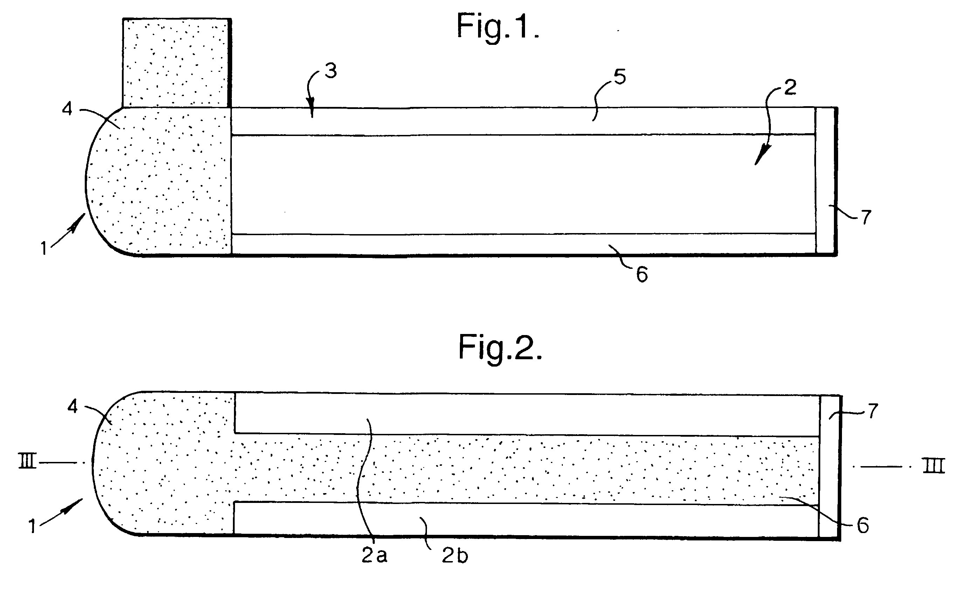

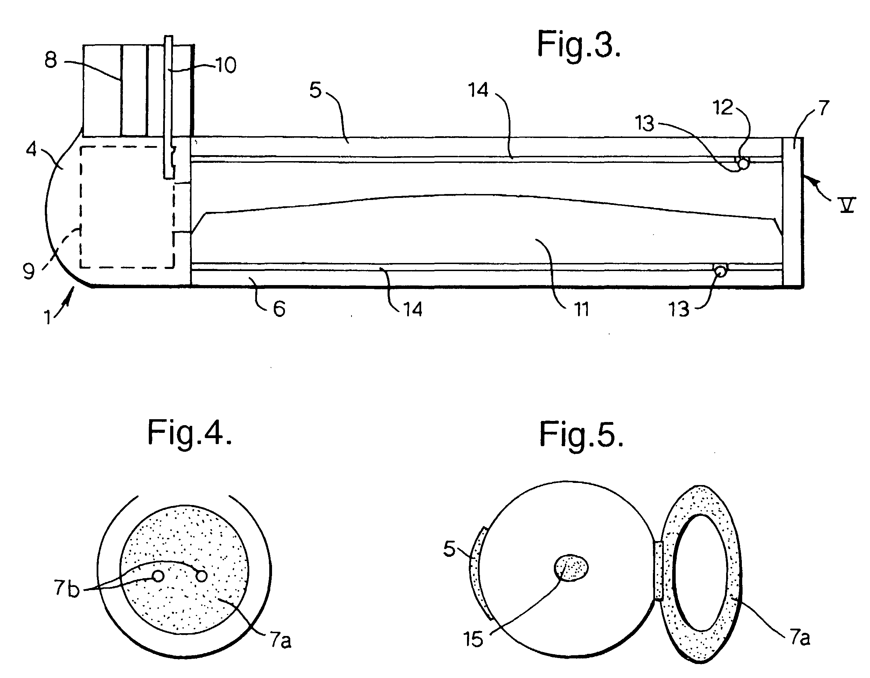

Referring to the drawings, and initially to the first embodiment of the invention shown in Figures 1 to 5, a handle is indicated generally by the reference numeral 1 and includes a hollow tubular handle member indicated generally by the reference numeral 2 and a holder indicated generally by the reference numeral 3. The holder 3 comprises a body portion 4, a pair of elongate arms 5 and 6, respectively, and an end cap 7. The arms 5 and 6 extend from the body portion 4 to the end cap 7 and serve to support the handle member 2, which is mounted for rotation within, and relative to, the holder 3. Each of the arms 5 and 6 has a curved profile in cross-section (see the arm 5 in Fig. 5), and extends along the sides of the handle member 2 leaving an upper portion 2a and a lower portion 2b of the handle member exposed. The end cap 7 comprises a hinged door 7a which can be locked shut by means of a lock 7b (as shown in Fig. 4) or opened (as shown in Fig. 5) to provide access to the handle member 2 and/or to allow removal of the handle member from the holder 3.

The body portion 4 is secured to one end portion of a spindle 8 (only a part of which is shown) which extends out of the body portion through a connecting plate (not shown) and, when the handle is used on a lavatory cistern, into a cistern (not shown). The spindle 8 links the handle 1 with an actuating mechanism within the cistern, depression of the free end of the handle (furthest from the body portion 4) causing rotation of the spindle, and hence actuation of a flushing mechanism within the cistern.

The body portion 4 also houses a clockwork motor 9 (indicated schematically in broken lines in Fig. 3) arranged in operation to rotate the handle member 2, one end of which is secured to the clockwork motor. A push rod 10 engages a ratchet (not shown) on the clockwork motor 9 to prevent operation thereof. The other end of the push rod 10 cooperates with a bevelled cam (not shown) on the connecting plate, so that when the handle 1 is depressed the cam causes the push rod to release the ratchet and the clockwork motor is allowed to rotate the handle member 2 by one turn. A legend "Clean" on the handle member 2 is so located as to appear on the upper part 2a of the handle member between the arms 5 and 6 each time the handle member has finished its turn.

The handle member 2 contains a disinfectant gel 11 maintained under a pressure slightly greater than atmospheric pressure (for example 2 bar). The handle member 2 is made from a flexible material and is formed with outlets 12 in its walls in the form of Bunsen holes or slits. The outlets 12 are normally closed. Protrusions 13 are provided on the inner surfaces of one or both of the arms 5 and 6 in positions aligned with the outlets 12. The protrusions 13 are such that they engage the handle member 2 in the regions of the outlets 12 on rotation of the handle member relative to the holder, and deform the handle member 2 in those regions causing the outlets to open and disinfectant gel to be discharged from the outlets. Plastic felt strips 14 secured to the inner surfaces of one or both of the arms 5 and 6 are provided to spread disinfectant discharged from the outlets 12 over the outer surface of the handle member 2 on rotation.

In operation, a user depresses the handle 1 by contact with the upper portion 2a of the handle member 2. Depression of the handle 1 causes the spindle 8 to rotate and the flushing mechanism of the cistern to be activated. At the same time, the cam on the connecting plate causes the push rod 10 to disengage the ratchet, allowing the clockwork motor 9 to rotate the handle member 2 by a single turn. As the handle member 2 rotates, the protrusions 13 cause the outlets 12 to open and discharge disinfectant gel onto the surface of the handle member. The disinfectant gel is spread over the surface of the handle member 2 in a thin film by the plastic felt strips 14. On completing a single turn of the handle member 12, the push rod 10 engages the ratchet and stops further operation of the motor 9. The legend "Clean" appears on the upper portion 2a of the handle member 2. The disinfectant gel acts to clean the handle member 2 of harmful bacteria for the next user.

When required, for example, at periodic intervals, access can be gained to the handle member 2 by opening the door 7a for complete removal and replacement of the tubular member by detaching from the clockwork motor 9 and withdrawal from the holder 3. Alternatively, the tubular member can remain *in situ* and be refilled with disinfectant using an injector device similar to a grease gun inserted into a nipple 15 in the end of the handle member 2 (see Fig. 5). At the same time, the clockwork motor 9 can be wound by rotating the handle member 2.

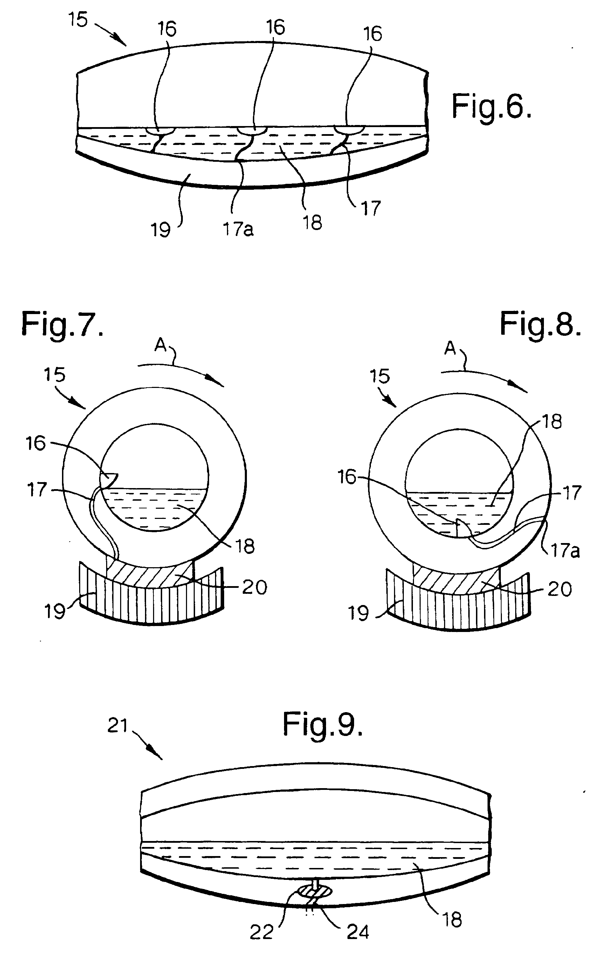

In a second embodiment of the handle 1, the handle member 2 is replaced by a hollow tubular handle member, indicated generally by the reference numeral 15, as shown in Figures 6 to 8. The handle member 15 includes, for example, three, spoon- or cup-shaped receptacles 16 that are attached to the inner surface of the handle member, or are formed integrally with the wall of the handle member. The interior of each receptacle 16 is in fluid communication with a respective drain tube 17 which passes through the wall of the handle member 15 to a discharge port 17a on the outer surface of the handle member. The receptacles 16, drain tubes 17 and discharge ports 17a together form discharging means for the handle member 15. The handle member 15 contains cleaning fluid 18 and the receptacles 16 are positioned so that, when the handle member is in a parked position, as shown in Figure 7, the receptacles are above the normal full level of the cleaning fluid 18 in the handle member. The handle member 15 is supported in the holder 3 and connected to the clockwork motor 9 in a similar manner to the handle member 2 except that the holder has only one arm 19 extending beneath the handle member 15. An absorbent pad 20 of sponge material is positioned between the handle member 15 and the arm 19 to provide distributing means for the cleaning fluid 18.

In operation, on actuation of the handle incorporating the handle member 15, the handle member is rotated by the clockwork motor 9 as in the first embodiment of the invention, the direction of rotation being indicated by arrow A in Figures 7 and 8. The receptacles 16 and the drain tubes 17 rotate with the handle member 15 and, as shown in Figure 8, the receptacles pass through the cleaning fluid 18, so that they are filled with cleaning fluid. When the handle member 15 has completed one revolution, the receptacles 16 are once again in the parked position (as shown in Figure 7) and the cleaning fluid from each receptacle flows through the drain tubes 17 out of the discharge ports 17a onto the pad 20 where it is absorbed. In that manner, a measured quantity of disinfectant is passed to the exterior of the handle member, that measured quantity being determined by the capacity and number of spoon- or cup-shaped receptacles 16 used. In that way, the absorbent pad 20 is kept replenished with the cleaning agent and, on each rotation of the handle member 15, the absorbent pad spreads the cleaning fluid over the outer surface of the handle member. The absorbent pad 20 can also prevent dripping or wastage of cleaning fluid 18 when the handle member is stationary in the parked position.

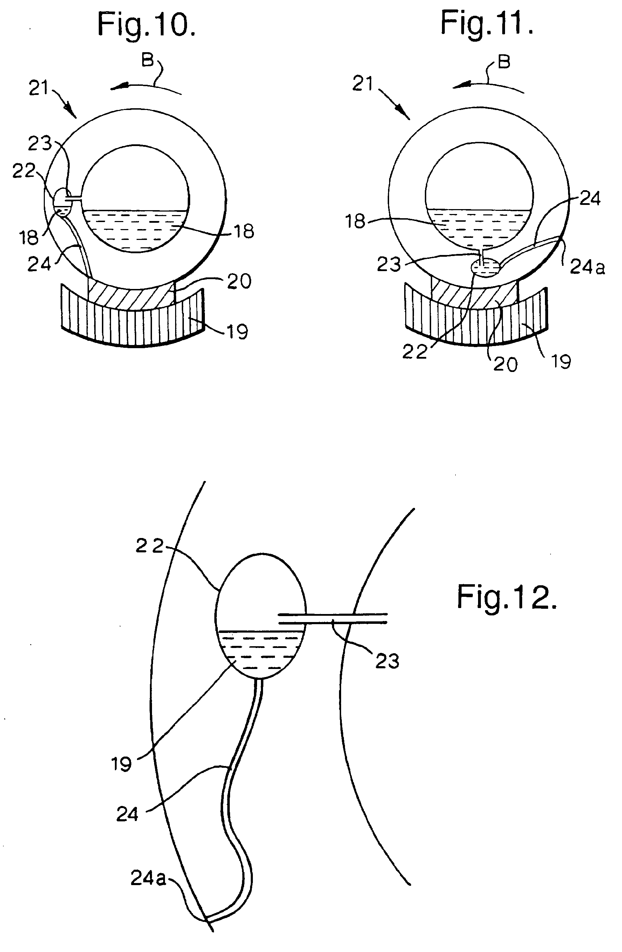

In a third embodiment of the handle 1, the handle member 2 is replaced by a hollow tubular handle member, indicated generally by the reference numeral 21 as shown in Figures 9 to 12. The handle member 21 includes a number of cavities 22 formed in the wall of the handle member. Each cavity 22 is in fluid communication with the interior of the handle member 21 via a filling tube 23 and is in fluid communication with a discharge port 24a on the outer surface of the handle member 21 via a drain tube 24 which passes through the wall of the handle member 21. The respective cavities 22, drain tubes 24 and discharge ports 24a together form discharging means for the handle member 21. The handle member 21 contains cleaning fluid 18 and the cavities 22 are positioned so that, when the handle member is in a parked position, as shown in Figure 10, the filling tubes 23 are above the full level of the cleaning fluid 18 in the handle member. The handle member 21 is supported in the holder 3 and connected to the clockwork motor 9 in a similar manner to handle member 15 with the arm 19 extending beneath the handle member, and the absorbent pad 20 of sponge material positioned between the handle member 21 and the arm.

In operation, on actuation of the handle incorporating the handle member 21, the handle member is rotated by the clockwork motor 9 as in the first and second embodiments of the invention, the direction of rotation being indicated by the arrow B in Figures 10 and 11. The cavities 22, filling tubes 23 and drain tubes 24 are rotated with the handle member 21 and, as shown in Figure 11, the cavities pass under the reservoir of cleaning fluid 18 in the handle member, so that cleaning fluid enters the cavities via the filling tubes. The filling tube 23 protrudes into both the cavity 22 and the interior of the handle member so that a quantity of cleaning fluid 18 is retained in the handle member 21 throughout the rotation cycle. When the handle member 21 has completed one revolution, the cavities 22 are once again in the parked position, as shown in Figure 10, and the cleaning fluid 18 from each cavity passes through the drain tubes 24 out of the discharge ports 24a and onto the absorbent pad 20. The absorbent pad 20 spreads the cleaning fluid over the outer surface of the handle member 21 as described with reference to the second embodiment. The absorbent pad 20 can also prevent dripping or wastage of cleaning fluid 18 when the handle member is stationary.

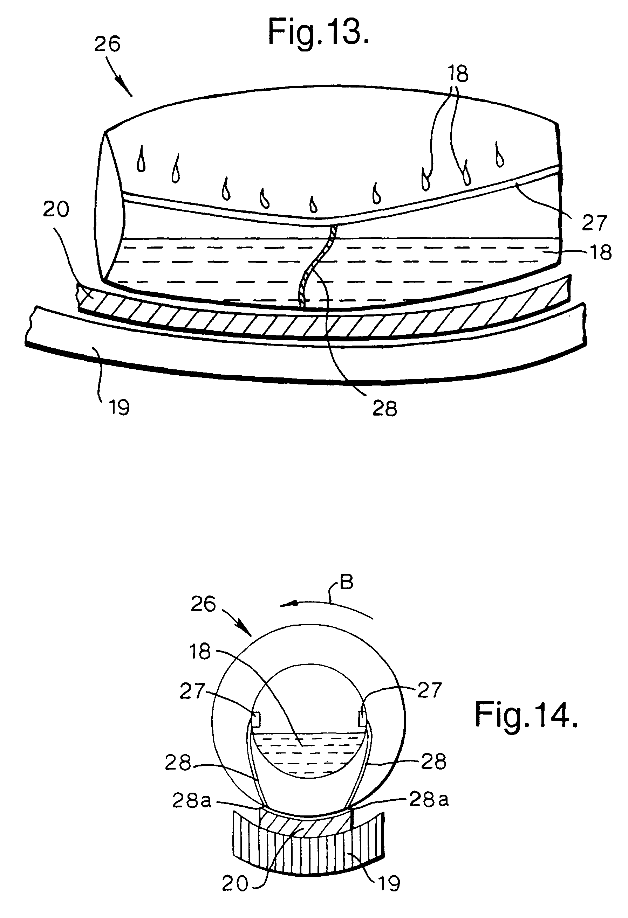

With reference to Figures 13 and 14, in a fourth embodiment of the invention the handle 1 includes a hollow tubular handle member, indicated generally by the reference numeral 26. The handle member 26 includes one or more gutters 27 extending longitudinally from one end of the handle member to the other. The gutters 27 are sloped so that any liquid that is collected flows to the lowest point of the gutter midway along the length of the handle member 26. A drain tube 28 is connected at the lowest point of the or each gutter 27, each drain tube connecting the respective gutter with a respective discharge port 28a on the outer surface of the handle member. The gutters, drain tubes and drain discharge ports together form discharging means for the handle member 26. The handle member 26 contains cleaning fluid 18 and the gutters 27 are positioned so that, when the handle member is in the parked position the gutters are above the full level of the cleaning fluid. The handle member 26 is connected to the clockwork motor 9 in a similar manner to handle members 2, 15 and 21. The arm 19 and the absorbent pad 20 are positioned beneath the handle member 26.

In operation, on actuation of the handle incorporating the handle member 26, the handle member is rotated by the clockwork motor 9 as in the earlier embodiments of the invention, the direction of rotation being indicated by the arrow B in Figure 14. The gutters 27 and drain tubes 28 are rotated with the handle member 26. When the handle member returns to the parked position, a quantity of cleaning fluid remains on the inner surface of the handle member 26 above the level of the gutters 27. That cleaning fluid runs down into the gutters and is discharged onto absorbent pad 20 via the drain tube 28 and the drain discharge port 28a. The absorbent pad 20 spreads the cleaning fluid 18 over the outer surface of the handle member 26 on the next rotation cycle as previously described. The absorbent pad can also prevent dripping and wastage of cleaning fluid 18 when the handle member 26 is in the parked position.

Although the handle 1 has been described above as adapted for use on a lavatory cistern, it will be appreciated that the handle of the invention can readily be adapted for use in other applications, for example, doors to toilets, kitchen doors and appliances, and also medical appliances and other uses in hospital environments. The clockwork motor 9 could also be replaced by an electric motor.

## Claims

1. A handle which comprises:
a hollow handle member the outer surface of which, in use, is contacted by the hand of an operator on actuation of the handle, the handle member being arranged to contain a cleaning agent and having means for discharging cleaning agent;
a holder for the handle member, the handle member being mounted for rotation about its longitudinal axis relative to the holder, the discharging means of the handle member being arranged to allow cleaning agent to be discharged from the interior of the handle member to the exterior upon such relative rotation and/or to be discharged subsequent to such relative rotation;
means for rotating the handle member about its longitudinal axis relative to the holder in response to the actuation of the handle; and
means for distributing cleaning agent discharged by the discharging means over the outer surface of the handle member upon relative rotation of the handle member and the holder.

2. A handle as claimed in claim 1, wherein the means for distributing cleaning agent over the outer surface of the handle member is arranged to absorb or otherwise retain cleaning fluid discharged by the discharging means for distributing over the outer surface of the handle member upon relative rotation.

3. A handle as claimed in claim 1 or claim 2, wherein the holder comprises one or more supporting arms for the handle member, the or each arm extending longitudinally relative to the handle member.

4. A handle as claimed in claim 3, wherein the means for distributing cleaning agent over the outer surface of the handle member is located between one or more of the arms and the handle member.

5. A handle as claimed in any one of claims 1 to 4, wherein the discharging means is in the form of one or more outlets in the handle member and the holder is arranged to open the or each outlet on rotation of the handle member relative to the holder.

6. A handle as claimed in claim 5, wherein the or each outlet is arranged to be opened by deformation of the region of the handle member in the area immediately surrounding the outlet and the holder is arranged to deform the said region on relative rotation of the handle member.

7. A handle as claimed in claim 6, wherein the holder comprises one or more arms, the arms extending longitudinally relative to the handle member and having one or more protrusions spaced along the arms, each of the protrusions being positioned so as to engage the handle member in the region of one of the outlets when the handle member rotates relative to the holder to cause the said deformation.

8. A handle as claimed in any one of claims 1 to 4, wherein the discharging means of the handle member comprises collecting means for collecting cleaning agent on relative rotation of the handle member and the holder, the discharging means being arranged to dispense the collected cleaning agent to the distributing means.

9. A handle as claimed in claim 8, wherein the discharging means further comprises one or more ducts or conduits arranged to allow collected cleaning agent to pass through the handle member to one or more discharge ports on its outer surface.

10. A handle as claimed in claim 9, wherein the handle member is arranged to rotate from a parked position by one or more whole turns relative to the holder on actuation of the handle, and the or each discharge port is arranged to coincide with the distributing means in the said parked position.

11. A handle as claimed in any of claims 8 to 10, wherein the collecting means comprises one or more receptacles on the inside of the handle member.

12. A handle as claimed in any one of claims 8 to 10 wherein the collecting means comprises one or more cavities in the handle member.

13. A handle as claimed in claim 12, wherein each cavity is provided with a filling tube which protrudes from the interior of the handle member into the cavity and is arranged to allow cleaning agent into the cavity but prevent it from returning to the interior of the handle member during rotation of the handle member relative to the holder.

14. A handle as claimed in any one of claims 8 to 10, wherein the collecting means comprises one or more gutters on the inside of the handle member, the or each gutter being arranged to collect cleaning agent remaining on the inside surface of the handle member after relative rotation.

15. A handle as claimed in any one of claims 1 to 14, wherein the means for rotating the handle member in response to the actuation of the handle comprises a clockwork motor.

16. A handle as claimed in any one of claims 1 to 14, wherein the means for rotating the handle member in response to the actuation of the handle comprises an electric motor.

17. A handle as claimed in any one of claims 1 to 16, wherein the cleaning agent comprises isopropyl alcohol.

18. A handle as claimed in any one of claims 1 to 17, wherein the hollow handle member is removable from the holder for refilling or replacement.

19. A handle as claimed in any one of claims 1 to 18, wherein the handle member is provided with a non-return valve so arranged as to allow the handle member to be supplied with cleaning agent.

20. A handle as claimed in claim 19, which further comprises a lockable end cap at one end of the handle member for covering the non-return valve.
